# EUROPEAN PATENT SPECIFICATION

(11) **EP 1 315 745 B1**
(45) Date of publication and mention of the grant of the patent: **14.06.2006**
(21) Application number: 01971961.6
(22) Date of filing: 24.08.2001
(51) Int. Cl.: C07K 14/34, C12N 9/02, C12N 15/52, C12N 1/20, C12R 1/15, C12P 13/08

(54) **RECOMBINANT CORYNEFORM BACTERIA OVEREXPRESSING THE GLYCERALDEHYDE-3-PHOSPHATE DEHYDROGENASE -2 GENE , AND THEIR USE IN L-LYSINE PRODUCTION**
REKOMBINANTE CORYNEFORMBAKTERIE DIE GLYCERALDEHYDE-3-PHOSPHATE DEHYDROGENASE -2 ÜBEREXPRIMIEREN , UND VERFAHREN ZUR HERSTELLUNG VON L-LYSINE
BACTERIES CORYNEFORMES RECOMBINANTES SUREXPRIMANT LA GLYCERALDEHYDE-3-PHOSPHATE DEHYDROGENASE -2, ET LEUR UTILISATION POUR LA PRODUCTION DE LA L-LYSINE

(30) Priority: 09.09.2000 DE 10044754; 28.07.2001 DE 10136985
(43) Date of publication of application: 04.06.2003
(73) Proprietor: Degussa AG, 40474 Düsseldorf (DE)
(72) Inventor: BATHE, Brigitte, 33154 Salzkotten (DE); HANS, Stephan, 49078 Osnabrück (DE); PFEFFERLE, Walter, 33790 Halle (Westf.) (DE)
(86) International application number: PCT/EP2001/009785
(87) International publication number: WO 2002/020542

(56) References cited:
- EP-A- 0 806 480
- EP-A- 1 108 790
- WO-A-01/00844
- EIKMANNS BERNHARD J: "Identification, sequence analysis, and expression of a Corynebacterium glutamicum gene cluster encoding the three glycolytic enzymes glyceraldehyde-3-phosphate dehydrogenase, 3-phosphoglycerate kinase, and triosephosphate isomerase." JOURNAL OF BACTERIOLOGY, vol. 174, no. 19, 1992, pages 6076-6086, XP000979491 ISSN: 0021-9193
- DATABASE TREMBL [Online] 1 August 1998 (1998-08-01) REEVES,C.D., ET AL.: " GLYCERALDEHYDE-3-PHOSPHATE DEHYDROGENASE HOMOLOG." retrieved from EBI Database accession no. O68923 XP002190186

## Description

### Field of the Invention

The invention provides nucleotide sequences from coryneform bacteria which code for the gap2 gene and a process for the fermentative preparation of amino acids using bacteria in which the endogenous gap2 gene is enhanced.

### Prior Art

L-Amino acids, in particular L-lysine, are used in human medicine and in the pharmaceuticals industry, in the foodstuffs industry and especially in animal nutrition.

It is known that amino acids are prepared by fermentation from strains of coryneform bacteria, in particular Corynebacterium glutamicum. Because of their great importance, work is constantly being undertaken to improve the preparation processes. Improvements to the process can relate to fermentation measures, such as, for example, stirring and supply of oxygen, or the composition of the nutrient media, such as, for example, the sugar concentration during the fermentation, or the working up to the product form by, for example, ion exchange chromatography, or the intrinsic output properties of the microorganism itself.

Methods of mutagenesis, selection and mutant selection are used to improve the output properties of these microorganisms. Strains which are resistant to antimetabolites or are auxotrophic for metabolites of regulatory importance and produce amino acids are obtained in this manner.

Methods of the recombinant DNA technique have also been employed for some years for improving the strain of Corynebacterium strains which produce L-amino acids, by amplifying individual amino acid biosynthesis genes and investigating the effect on the amino acid production.

In EP 0 806 480 A2 a Streptomyces frenolicin gene cluster is disclosed encoding enzymes which catalyze the biosynthesis of frenocilius in prokaryotic cells.

The gene cluster includes a gene with high similarity to those encoding glyceraldehyde-3-phosphate dehydrogenase from a variety of organisms.

Database Trembl [Online] 1. August 1988 (1988-08-01) Reeves et al. discloses a glyceraldehyde-3-phosphate dehydrogenase homolog. from Streptomyces roseofulvus.

WO 01/00844 A and EP 1 108 790 A1 disclose sequences of novel polynucleotides of Corynebacterium glutamicum but offer no technical features beyond a vague statement that such nucleic acid molecules can be put to a variety of general uses.

### Object of the Invention

The inventors had the object of providing new measures for improved fermentative preparation of amino acids.

### Summary of the Invention

Where L-amino acids or amino acids are mentioned in the following, this means one or more amino acids, including their salts, chosen from the group consisting of L-asparagine, L-threonine, L-serine, L-glutamate, L-glycine, L-alanine, L-cysteine, L-valine, L-methibnine, L-isoleucine, L-leucine, L-tyrosine, L-phenylalanine, L-histidine, L-lysine, L-tryptophah and L-arginine. L-Lysine is particularly preferred.

Where L-lysine or lysine are mentioned in the following, this means not only the bases but also the salts, such as e.g. lysine monohydrochloride or lysine sulfate.

The invention disclosed an isolated polynucleotide from coryneform bacteria, comprising a polynucleotide sequence which codes for the gap2 gene, chosen from the group consisting of
a) polynucleotide which is identical to the extent of at least 70% to a polynucleotide which codes for a polypeptide which comprises the amino acid sequence of SEQ ID No. 2,
b) polynucleotide which codes for a polypeptide which comprises an amino acid sequence which is identical to the extent of at least 70% to the amino acid sequence of SEQ ID No.2
c) polynucleotide which is complementary to the polynucleotides of a) or b), and
d) polynucleotide comprising at least 15 successive nucleotides of the polynucleotide sequence of a), b) or c),
the polypeptide preferably having the activity of glyceraldehyde 3-phosphate dehydrogenase 2.

The invention also discloses the above-mentioned polynucleotide, this preferably being a DNA which is capable of replication, comprising:
(i) the nucleotide sequence shown in SEQ ID No. 1, or
(ii) at least one sequence which corresponds to sequence (i) within the range of the degeneration of the genetic code, or
(iii) at least one sequence which hybridizes with the sequence complementary to sequence (i) or (ii), and optionally
(iv) sense mutations of neutral function in (i).

The invention also discloses
A polynucleotide, in particular DNA, which is capable of replication and comprises the nucleotide sequence as shown in SEQ ID No. 1;
a polynucleotide which codes for a polypeptide which comprises the amino acid sequence as shown in SEQ ID No. 2;
a vector containing the polynucleotide according to the invention, in particular a shuttle vector or plasmid vector, and
coryneform bacteria which contain the vector or in which the endogenous gap2 gene is enhanced.

The invention also discloses polynucleotides which substantially comprise a polynucleotide sequence, which are obtainable by screening by means of hybridization of a corresponding gene library of a coryneform bacterium, which comprises the complete gene or parts thereof, with a probe which comprises the sequence of the polynucleotides according to the invention according to SEQ ID No. 1 or a fragment thereof, and isolation of the polynucleotide sequence mentioned.

### Detailed Description of the Invention

Polynucleotides which comprise the sequences according to the invention are disclosed as hybridization probes for RNA, cDNA and DNA, in order to isolate, in the full length, nucleic acids or polynucleotides or genes which code for glyceraldehyde 3-phosphate dehydrogenase 2 or to isolate those nucleic acids or polynucleotides or genes which have a high similarity of sequence with that of the gap2 gene. They are also disclosed for incorporation into so-called "arrays", "micro arrays" or "DNA chips" in order to detect and to determine the corresponding polynucleotides.

Polynucleotides which comprise the sequences according to the invention are furthermore disclosed as primers with the aid of which DNA of genes which code for glyceraldehyde 3-phosphate dehydrogenase 2 can be prepared by the polymerase chain reaction (PCR).

Such oligonucleotides which serve as probes or primers comprise at least 25, 26, 27, 28, 29 or 30, preferably at least 20, 21, 22, 23 or 24, very particularly preferably at least 15, 16, 17, 18 or 19 successive nucleotides. Oligonucleotides which have a length of at least 31, 32, 33, 34, 35, 36, 37, 38, 39 or 40, or at least 41, 42, 43, 44, 45, 46, 47, 48, 49 or 50 nucleotides are also suitable. Oligonucleotides with a length of at least 100, 150, 200, 250 or 300 nucleotides are optionally also suitable. "Isolated" means separated out of its natural environment.

"Polynucleotide" in general relates to polyribonucleotides and polydeoxyribonucleotides, it being possible for these to be non-modified RNA or DNA or modified RNA and DNA.

The polynucleotides according to the invention include a polynucleotide according to SEQ ID No. 1 or a fragment prepared therefrom and also those which are at least in particular 70% to 80%, preferably at least 81% to 85%, particularly preferably at least 86% to 90%, and very particularly preferably at least 91%, 93%, 95%, 97% or 99% identical to the polynucleotide according to SEQ ID No. 1 or a fragment prepared therefrom.

"Polypeptides" are understood as meaning peptides or proteins which comprise amino acids bonded via two or more peptide bonds.

The polypeptides according to the invention include a polypeptide according to SEQ ID No. 2, in particular those with the biological activity of glyceraldehyde 3-phosphate dehydrogenase 2 and also those which are at least 70% to 80%, preferably at least 81% to 85%, particularly preferably at least 86% to 90%, and very particularly preferably at least 91%, 93%, 95%, 97% or 99% identical to the polypeptide according to SEQ ID No. 2 and have the activity mentioned.

The invention furthermore relates to a process for the fermentative preparation of amino acids chosen from the group consisting of L-asparagine, L-threonine, L-serine, L-glutamate, L-glycine, L-alanine, L-cysteine, L-valine, L-methionine, L-isoleucine, L-leucine, L-tyrosine, L-phenylalanine, L-histidine, L-lysine, L-tryptophan and L-arginine using coryneform bacteria which in particular already produce amino acids and in which the nucleotide sequences which code for the gap2 gene are enhanced, in particular over-expressed.

The term "enhancement" in this connection describes the increase in the intracellular activity of one or more enzymes in a microorganism which are coded by the corresponding DNA, for example by increasing the number of copies of the gene or genes, using a potent promoter or using a gene which codes for a corresponding enzyme having a high activity, and optionally combining these measures.

By enhancement measures, in particular over-expression, the activity or concentration of the corresponding protein is in general increased by at least 10%, 25%, 50%, 75%, 100%, 150%, 200%, 300%, 400% or 500%, up to a maximum of 1000% or 2000%, based on that of the wild-type protein or the activity or concentration of the protein in the starting microorganism.

The microorganisms which the present invention provides can produce L-amino acids from glucose, sucrose, lactose, fructose, maltose, molasses, starch, cellulose or from glycerol and ethanol. They can be representatives of coryneform bacteria, in particular of the genus Corynebacterium. Of the genus Corynebacterium, there may be mentioned in particular the species Corynebacterium glutamicum, which is known among experts for its ability to produce L-amino acids.

Suitable strains of the genus Corynebacterium, in particular the species Corynebacterium glutamicum (C. glutamicum), are in particular the known wild-type strains
Corynebacterium glutamicum ATCC13032
Corynebacterium acetoglutamicum ATCC15806
Corynebacterium acetoacidophilum ATCC13870
Corynebacterium thermoaminogenes FERM BP-1539
Corynebacterium melassecola ATCC17965
Brevibacterium flavum ATCC14067
Brevibacterium lactofermentum ATCC13869 and
Brevibacterium divaricatum ATCC14020
and L-amino acid-producing mutants or strains prepared therefrom.

The new gap2 gene from C. glutamicum which codes for the enzyme glyceraldehyde 3-phosphate dehydrogenase 2 (EC 1.2.1.12) has been isolated.

To isolate the gap2 gene or also other genes of C. glutamicum, a gene library of this microorganism is first set up in Escherichia coli (E. coli). The setting up of gene libraries is described in generally known textbooks and handbooks. The textbook by Winnacker: Gene und Klone, Eine Einführung in die Gentechnologie (Verlag Chemie, Weinheim, Germany, 1990), or the handbook by Sambrook et al.: Molecular Cloning, A Laboratory Manual (Cold Spring Harbor Laboratory Press, 1989) may be mentioned as an example. A well-known gene library is that of the E. coli K-12 strain W3110 set up in λ vectors by Kohara et al. (Cell 50, 495 -508 (1987)). Bathe et al. (Molecular and General Genetics, 252:255-265, 1996) describe a gene library of C. glutamicum ATCC13032, which was set up with the aid of the cosmid vector SuperCos I (Wahl et al., 1987, Proceedings of the National Academy of Sciences, USA, 84:2160-2164) in the E. coli K-12 strain NM554 (Raleigh et al., 1988, Nucleic Acids Research 16:1563-1575).

Börmann et al. (Molecular Microbiology 6(3), 317-326)) (1992)) in turn describe a gene library of C. glutamicum ATCC13032 using the cosmid pHC79 (Hohn and Collins, Gene 11, 291-298 (1980)).

To prepare a gene library of C. glutamicum in E. coli it is also possible to use plasmids such as pBR322 (Bolivar, Life Sciences, 25, 807-818 (1979)) or pUC9 (Vieira et al., 1982, Gene, 19:259-268). Suitable hosts are, in particular, those E. coli strains which are restriction- and recombination-defective. An example of these is the strain DH5αmcr, which has been described by Grant et al. (Proceedings of the National Academy of Sciences USA, 87 (1990) 4645-4649). The long DNA fragments cloned with the aid of cosmids can in turn be subcloned in the usual vectors suitable for sequencing and then sequenced, as is described e.g. by Sanger et al. (Proceedings of the National Academy of Sciences of the United States of America, 74:5463-5467, 1977).

The new DNA sequence of C. glutamicum which codes for the gap2 gene and which, as SEQ ID No. 1, is a constituent of the present invention has been found. The amino acid sequence of the corresponding protein has furthermore been derived from the present DNA sequence by the methods described above. The resulting amino acid sequence of the gap2 gene product is shown in SEQ ID No. 2. It is known that enzymes endogenous to the host can split off the N-terminal amino acid methionine or formylmethionine of the protein formed.

Coding DNA sequences which result from SEQ ID No. 1 by the degeneracy of the genetic code are also a constituent of the invention. In the same way, DNA sequences which hybridize with SEQ ID No. 1 or parts of SEQ ID No. 1 are a constituent of the invention. Conservative amino acid exchanges, such as e.g. exchange of glycine for alanine or of aspartic acid for glutamic acid in proteins, are furthermore known among experts as "sense mutations" which do not lead to a fundamental change in the activity of the protein, i.e. are of neutral function. Such mutations are also called, inter alia, neutral substitutions. It is furthermore known that changes on the N and/or C terminus of a protein cannot substantially impair or can even stabilize the function thereof. Information in this context can be found by the expert, inter alia, in Ben-Bassat et al. (Journal of Bacteriology 169:751-757 (1987)), in O'Regan et al. (Gene 77:237-251 (1989)), in Sahin-Toth et al. (Protein Sciences 3:240-247 (1994)), in Hochuli et al. (Bio/Technology 6:1321-1325 (1988)) and in known textbooks of genetics and molecular biology. Amino acid sequences which result in a corresponding manner from SEQ ID No. 2 are also a constituent of the invention.

In the same way, DNA sequences which hybridize with SEQ ID No. 1 or parts of SEQ ID No. 1 are a constituent of the invention. Finally, DNA sequences which are prepared by the polymerase chain reaction (PCR) using primers which result from SEQ ID No. 1 are a constituent of the invention. Such oligonucleotides typically have a length of at least 15 nucleotides.

Instructions for identifying DNA sequences by means of hybridization can be found by the expert, inter alia, in the handbook "The DIG System Users Guide for Filter Hybridization" from Boehringer Mannheim GmbH (Mannheim, Germany, 1993) and in Liebl et al. (International Journal of Systematic Bacteriology (1991) 41: 255-260). The hybridization takes place under stringent conditions, that is to say only hybrids in which the probe and target sequences, i. e. the polynucleotides treated with the probe, are at least 70 % identical are formed. It is known that the stringency of the hybridization, including the washing steps, is influenced or determined by varying the buffer composition, the temperature and the salt concentration. The hybridization reaction is preferably carried out under a relatively low stringency compared with the washing steps (Hybaid Hybridisation Guide, Hybaid Limited, Teddington, UK, 1996).

A 5x SSC buffer at a temperature of approx. 50 - 68°C, for example, can be employed for the hybridization reaction. Probes can also hybridize here with polynucleotides which are less than 70% identical to the sequence of the probe. Such hybrids are less stable and are removed by washing under stringent conditions. This can be achieved, for example, by lowering the salt concentration to 2x SSC and optionally subsequently 0.5x SSC (The DIG System User's Guide for Filter Hybridisation, Boehringer Mannheim, Mannheim, Germany, 1995) a temperature of approx. 50 - 68°C being established. It is optionally possible to lower the salt concentration to 0.1x SSC. Polynucleotide fragments which are, for example, at least 70% or at least 80% or at least 90% to 95% identical to the sequence of the probe employed can be isolated by increasing the hybridization temperature stepwise from 50 to 68°C in steps of approx. 1 - 2°C. Further instructions on hybridization are obtainable on the market in the form of so-called kits (e.g. DIG Easy Hyb from Roche Diagnostics GmbH, Mannheim, Germany, Catalogue No. 1603558).

Instructions for amplification of DNA sequences with the aid of the polymerase chain reaction (PCR) can be found by the expert, inter alia, in the handbook by Gait: Oligonucleotide Synthesis: A Practical Approach (IRL Press, Oxford, UK, 1984) and in Newton and Graham: PCR (Spektrum Akademischer Verlag, Heidelberg, Germany, 1994).

It has been found that coryneform bacteria produce amino acids in an improved manner after over-expression of the gap2 gene.

To achieve an over-expression, the number of copies of the corresponding genes can be increased, or the promoter and regulation region or the ribosome binding site upstream of the structural gene can be mutated. Expression cassettes which are incorporated upstream of the structural gene act in the same way. By inducible promoters, it is additionally possible to increase the expression in the course of fermentative amino acid production. The expression is likewise improved by measures to prolong the life of the m-RNA. Furthermore, the enzyme activity is also increased by preventing the degradation of the enzyme protein. The genes or gene constructs can either be present in plasmids with a varying number of copies, or can be integrated and amplified in the chromosome. Alternatively, an over-expression of the genes in question can furthermore be achieved by changing the composition of the media and the culture procedure.

Instructions in this context can be found by the expert, inter alia, in Martin et al. (Bio/Technology 5, 137-146 (1987)), in Guerrero et al. (Gene 138, 35-41 (1994)), Tsuchiya and Morinaga (Bio/Technology 6, 428-430 (1988)), in Eikmanns et al. (Gene 102, 93-98 (1991)), in European Patent Specification 0 472 869, in US-Patent 4,601,893, in Schwarzer and Pühler (Bio/Technology 9, 84-87 (1991)), Reinscheid et al. (Applied and Environmental Microbiology 60, 126-132 (1994)), in LaBarre et al. (Journal of Bacteriology 175, 1001-1007 (1993)), in Patent Application WO 96/15246, in Malumbres et al. (Gene 134, 15 - 24 (1993)), in Japanese Laid-Open Specification JP-A-10-229891, in Jensen and Hammer (Biotechnology and Bioengineering 58, 191-195 (1998)), in Makrides (Microbiological Reviews 60:512-538 (1996)) and in known textbooks of genetics and molecular biology.

By way of example, for enhancement the gap2 gene according to the invention was over-expressed with the aid of episomal plasmids. Suitable plasmids are those which are replicated in coryneform bacteria. Numerous known plasmid vectors, such as e.g. pZ1 (Menkel et al., Applied and Environmental Microbiology (1989) 64: 549-554), pEKExl (Eikmanns et al., Gene 102:93-98 (1991)) or pHS2-1 (Sonnen et al., Gene 107:69-74 (1991)) are based on the cryptic plasmids pHM1519, pBL1 or pGA1. Other plasmid vectors, such as e.g. those based on pCG4 (US-A 4,489,160), or pNG2 (Serwold-Davis et al., FEMS Microbiology Letters 66, 119-124 (1990)), or pAG1 (US-A 5,158,891), can be used in the same manner.

Plasmid vectors which are furthermore suitable are also those with the aid of which the process of gene amplification by integration into the chromosome can be used, as has been described, for example, by Reinscheid et al. (Applied and Environmental Microbiology 60, 126-132 (1994)) for duplication or amplification of the hom-thrB operon. In this method, the complete gene is cloned in a plasmid vector which can replicate in a host (typically E. coli), but not in C. glutamicum. Possible vectors are, for example, pSUP301 (Simon et al., Bio/technology 1, 784-791 (1983)), pK18mob or pK19mob (Schäfer et al., Gene 145, 69-73 (1994)), pGEM-T (Promega corporation, Madison, WI, USA), pCR2.1-TOPO (Shuman (1994). Journal of Biological Chemistry 269:32678-84; US-A 5,487,993), pCR®Blunt (Invitrogen, Groningen, Holland; Bernard et al., Journal of Molecular Biology, 234: 534-541 (1993)), pEM1 (Schrumpf et al., 1991, Journal of Bacteriology 173:4510-4516) or pBGS8 (Spratt et al.,1986, Gene 41: 337-342). The plasmid vector which contains the gene to be amplified is then transferred into the desired strain of C. glutamicum by conjugation or transformation. The method of conjugation is described, for example, by Schäfer et al. (Applied and Environmental Microbiology 60, 756-759 (1994)). Methods for transformation are described, for example, by Thierbach et al. (Applied Microbiology and Biotechnology 29, 356-362 (1988)), Dunican and Shivnan (Bio/Technology 7, 1067-1070 (1989)) and Tauch et al. (FEMS Microbiological Letters 123, 343-347 (1994)). After homologous recombination by means of a "cross over" event, the resulting strain contains at least two copies of the gene in question. In addition, it may be advantageous for the production of L-amino acids to enhance, in particular over-express one or more enzymes of the particular biosynthesis pathway, of glycolysis, of anaplerosis, of the citric acid cycle, of the pentose phosphate cycle, of amino acid export and optionally regulatory proteins, in addition to the gap2 gene.

Thus, for example, for the preparation of L-amino acids, in addition to enhancement of the gap2 gene, one or more endogenous genes chosen from the group consisting of
- the dapA gene which codes for dihydrodipicolinate synthase (EP-B 0 197 335),
- the gap gene which codes for glyceraldehyde 3-phosphate dehydrogenase (Eikmanns (1992), Journal of Bacteriology 174:6076-6086),
- the tpi gene which codes for triose phosphate isomerase (Eikmanns (1992), Journal of Bacteriology 174:6076-6086),
- the pgk gene which codes for 3-phosphoglycerate kinase (Eikmanns (1992), Journal of Bacteriology 174:6076-6086),
- the zwf gene which codes for glucose 6-phosphate dehydrogenase (JP-A-09224661),
- the pyc gene which codes for pyruvate carboxylase (DE-A 198 31 609),
- the mqo gene which codes for malate-quinone oxidoreductase (Molenaar et al., European Journal of Biochemistry 254, 395-403 (1998)),
- the lysC gene which codes for a feed-back resistant aspartate kinase (Accession No.P26512; EP-B-0387527; EP-A-0699759),
- the lysE gene which codes for lysine export (DE-A-195 48 222),
- the hom gene which codes for homoserine dehydrogenase (EP-A 0131171),
- the ilvA gene which codes for threonine dehydratase (Möckel et al., Journal of Bacteriology (1992) 8065-8072) or the ilvA(Fbr) allele which codes for a feed back resistant threonine dehydratase (Möckel et al., (1994) Molecular Microbiology 13: 833-842),
- the ilvBN gene which codes for acetohydroxy-acid synthase (EP-B 0356739),
- the ilvD gene which codes for dihydroxy-acid dehydratase (Sahm and Eggeling (1999) Applied and Environmental Microbiology 65: 1973-1979),
- the zwal gene which codes for the Zwa1 protein (DE: 19959328.0, DSM 13115)
- can be enhanced, in particular over-expressed.

It may furthermore be advantageous for the production of L-amino acids, in addition to the enhancement of the gap2 gene, for one or more genes chosen from the group consisting of:
- the pck gene which codes for phosphoenol pyruvate carboxykinase (DE 199 50 409.1; DSM 13047),
- the pgi gene which codes for glucose 6-phosphate isomerase (US 09/396,478; DSM 12969),
- the poxB gene which codes for pyruvate oxidase (DE: 1995 1975.7; DSM 13114),
- the zwa2 gene which codes for the Zwa2 protein (DE: 19959327.2, DSM 13113)
to be attenuated, in particular for the expression thereof to be reduced.

The term "attenuation" in this connection describes the reduction or elimination of the intracellular activity of one or more enzymes (proteins) in a microorganism which are coded by the corresponding DNA, for example by using a weak promoter or using a gene or allele which codes for a corresponding enzyme with a low activity or inactivates the corresponding gene or enzyme (protein), and optionally combining these measures.

By attenuation measures, the activity or concentration of the corresponding protein is in general reduced to 0 to 75%, 0 to 50%, 0 to 25%, 0 to 10% or 0 to 5% of the activity or concentration of the wild-type protein or of the activity or concentration of the protein in the starting microorganism.

In addition to over-expression of the gap2 gene it may furthermore be advantageous for the production of amino acids to eliminate undesirable side reactions (Nakayama: "Breeding of Aminoacid Producing Micro-organisms", in: Overproduction of Microbial Products, Krumphanzl, Sikyta, Vanek (eds.), Academic Press, London, UK, 1982).

The invention also provides the microorganisms prepared according to the invention, and these can be cultured continuously or discontinuously in the batch process (batch culture) or in the fed batch (feed process) or repeated fed batch process (repetitive feed process) for the purpose of production of amino acids. A summary of known culture methods is described in the textbook by Chmiel (Bioprozesstechnik 1. Einführung in die Bioverfahrenstechnik (Gustav Fischer Verlag, Stuttgart, 1984)) or in the textbook by Storhas (Bioreaktoren und periphere Einrichtungen (Vieweg Verlag, Braunschweig/Wiesbaden, 1994)).

The culture medium to be used must meet the requirements of the particular strains in a suitable manner. Descriptions of culture media for various microorganisms are contained in the handbook "Manual of Methods for General Bacteriology" of the American Society for Bacteriology (Washington D.C., USA, 1981).

Sugars and carbohydrates, such as e.g. glucose, sucrose, lactose, fructose, maltose, molasses, starch and cellulose, oils and fats, such as e.g. soya oil, sunflower oil, groundnut oil and coconut fat, fatty acids, such as e.g. palmitic acid, stearic acid and linoleic acid, alcohols, such as e.g. glycerol and ethanol, and organic acids, such as e.g. acetic acid, can be used as the source of carbon. These substances can be used individually or as a mixture.

Organic nitrogen-containing compounds, such as peptones, yeast extract, meat extract, malt extract, corn steep liquor, soya bean flour and urea, or inorganic compounds, such as ammonium sulfate, ammonium chloride, ammonium phosphate, ammonium carbonate and ammonium nitrate, can be used as the source of nitrogen. The sources of nitrogen can be used individually or as a mixture.

Phosphoric acid, potassium dihydrogen phosphate or dipotassium hydrogen phosphate or the corresponding sodium-containing salts can be used as the source of phosphorus. The culture medium must furthermore comprise salts of metals, such as e. g. magnesium sulfate or iron sulfate, which are necessary for growth. Finally, essential growth substances, such as amino acids and vitamins, can be employed in addition to the above-mentioned substances. Suitable precursors can moreover be added to the culture medium. The starting substances mentioned can be added to the culture in the form of a single batch, or can be fed in during the culture in a suitable manner.

Basic compounds, such as sodium hydroxide, potassium hydroxide, ammonia or aqueous ammonia, or acid compounds, such as phosphoric acid or sulfuric acid, can be employed in a suitable manner to control the pH of the culture. Antifoams, such as e.g. fatty acid polyglycol esters, can be employed to control the development of foam. Suitable substances having a selective action, such as e.g. antibiotics, can be added to the medium to maintain the stability of plasmids. To maintain aerobic conditions, oxygen or oxygen-containing gas mixtures, such as e.g. air, are introduced into the culture. The temperature of the culture is usually 20°C to 45°C and preferably 25°C to 40°C. Culturing is continued until a maximum of the desired product has formed. This target is usually reached within 10 hours to 160 hours.

Methods for the determination of L-amino acids are known from the prior art. The analysis can thus be carried out, for example, as described by Spackman et al. (Analytical Chemistry, 30, (1958), 1190) by ion exchange chromatography with subsequent ninhydrin derivation, or it can be carried out by reversed phase HPLC, for example as described by Lindroth et al. (Analytical Chemistry (1979) 51: 1167-1174).

The process according to the invention is used for fermentative preparation of amino acids.

The following microorganism was deposited as a pure culture on 18th July 2001 at the Deutsche Sammlung für Mikroorganismen und Zellkulturen (DSMZ = German Collection of Microorganisms and Cell Cultures, Braunschweig, Germany) in accordance with the Budapest Treaty:
- Escherichia coli DH5αmcr/pEC-K18mob2gap2exp as DSM 14407.

The present invention is explained in more detail in the following with the aid of embodiment examples.

The isolation of plasmid DNA from Escherichia coli and all techniques of restriction, Klenow and alkaline phosphatase treatment were carried out by the method of Sambrook et al. (Molecular Cloning. A Laboratory Manual (1989) Cold Spring Harbor Laboratory Press, Cold Spring Harbor, NY, USA). Methods for transformation of Escherichia coli are also described in this handbook.

The composition of the usual nutrient media, such as LB or TY medium, can also be found in the handbook by Sambrook et al.

### Example 1

### Preparation of a genomic cosmid gene library from Corynebacterium glutamicum ATCC 13032

Chromosomal DNA from Corynebacterium glutamicum ATCC 13032 was isolated as described by Tauch et al. (1995), Plasmid 33:168-179) and partly cleaved with the restriction enzyme Sau3AI (Amersham Pharmacia, Freiburg, Germany, Product Description Sau3AI, Code no. 27-0913-02). The DNA fragments were dephosphorylated with shrimp alkaline phosphatase (Roche Diagnostics GmbH, Mannheim, Germany, Product Description SAP, Code No. 1758250). The DNA of the cosmid vector SuperCos1 (Wahl et al. (1987) Proceedings of the National Academy of Sciences USA 84:2160-2164), obtained from Stratagene (La Jolla, USA, Product Description SuperCos1 Cosmid Vector Kit, Code no. 251301) was cleaved with the restriction enzyme XbaI (Amersham Pharmacia, Freiburg, Germany, Product Description XbaI, Code no. 27-0948-02) and likewise dephosphorylated with shrimp alkaline phosphatase.

Finally, the cosmid DNA was cleaved with the restriction enzyme BamHI (Amersham Pharmacia, Freiburg, Germany, Product Description BamHI, Code no. 27-0868-04). The cosmid DNA treated in this manner was mixed with the treated ATCC13032 DNA and the batch was treated with T4 DNA ligase (Amersham Pharmacia, Freiburg, Germany, Product Description T4-DNA-Ligase, Code no. 27-0870-04). The ligation mixture was then packed in phages with the aid of Gigapack II XL Packing Extract (Stratagene, La Jolla, USA, Product Description Gigapack II XL Packing Extract, Code no. 200217).

For infection of the E. coli strain NM554 (Raleigh et al. 1988, Nucleic Acid Research 16:1563-1575) the cells were taken up in 10 mM MgSO₄ and mixed with an aliquot of the phage suspension. The infection and titering of the cosmid library were carried out as described by Sambrook et al. (1989, Molecular Cloning: A laboratory Manual, Cold Spring Harbor), the cells being plated out on LB agar (Lennox, 1955, Virology, 1:190) with 100 mg/l ampicillin. After incubation overnight at 37°C, recombinant individual clones were selected.

### Example 2

### Isolation and sequencing of the gap2 gene

The cosmid DNA of an individual colony was isolated with the Qiaprep Spin Miniprep Kit (Product No. 27106, Qiagen, Hilden, Germany) in accordance with the manufacturer's instructions and partly cleaved with the restriction enzyme Sau3AI (Amersham Pharmacia, Freiburg, Germany, Product Description Sau3AI, Product-No. 27-0913-02). The DNA fragments were dephosphorylated with shrimp alkaline phosphatase (Roche Diagnostics GmbH, Mannheim, Germany, Product Description SAP, Product No. 1758250). After separation by gel electrophoresis, the cosmid fragments in the size range of 1500 to 2000 bp were isolated with the QiaExII Gel Extraction Kit (Product No. 20021, Qiagen, Hilden, Germany).

The DNA of the sequencing vector pZero-1, obtained from Invitrogen (Groningen, The Netherlands, Product Description Zero Background Cloning Kit, Product No. K2500-01), was cleaved with the restriction enzyme BamHI (Amersham Pharmacia, Freiburg, Germany, Product Description BamHI No. 27-0868-04). The ligation of the cosmid fragments in the sequencing vector pZero-1 was carried out as described by Sambrook et al. (1989, Molecular Cloning: A Laboratory Manual, Cold Spring Harbor), the DNA mixture being incubated overnight with T4 ligase (Pharmacia Biotech, Freiburg Germany). This ligation mixture was then electroporated (Tauch et al. 1994, FEMS Microbiol Letters, 123:343-7) into the E. coli strain DH5αMCR (Grant, 1990, Proceedings of the National Academy of Sciences U.S.A., 87:4645-4649) and plated out on LB agar (Lennox, 1955, Virology 1:190) with 50 mg/l zeocin.

The plasmid preparation of the recombinant clones was carried out with the Biorobot 9600 (Product No. 900200, Qiagen, Hilden, Germany). The sequencing was carried out by the dideoxy chain termination method of Sanger et al. (1977, Proceedings of the National Academy of Sciences U.S.A., 74:5463-5467) with modifications according to Zimmermann et al. (1990, Nucleic Acids Research, 18:1067). The "RR dRhodamin Terminator Cycle Sequencing Kit" from PE Applied Biosystems (Product No. 403044, Weiterstadt, Germany) was used. The separation by gel electrophoresis and analysis of the sequencing reaction were carried out in a "Rotiphoresis NF Acrylamide/Bisacrylamide" Gel (29:1) (Product No. A124.1, Roth, Karlsruhe, Germany) with the "ABI Prism 377" sequencer from PE Applied Biosystems (Weiterstadt, Germany).

The raw sequence data obtained were then processed using the Staden program package (1986, Nucleic Acids Research, 14:217-231) version 97-0. The individual sequences of the pZero1 derivatives were assembled to a continuous contig. The computer-assisted coding region analysis was prepared with the XNIP program (Staden, 1986, Nucleic Acids Research, 14:217-231).

The resulting nucleotide sequence is shown in SEQ ID No. 1. Analysis of the nucleotide sequence showed an open reading frame of 1440 base pairs, which was called the gap2 gene. The gap2 gene codes for a protein of 480 amino acids.

### Example 3

Preparation of a shuttle vector pEC-K18mob2gap2exp for enhancement of the gap2 gene in C. glutamicum

### 3.1 Cloning of the gap2 gene in the vector pCR®Blunt II

From the strain ATCC 13032, chromosomal DNA was isolated by the method of Eikmanns et al. (Microbiology 140: 1817-1828 (1994)). On the basis of the sequence of the gap2 gene known for C. glutamicum from example 2, the following oligonucleotides were chosen for the polymerase chain reaction (see also SEQ ID No. 3 and SEQ ID No. 4):
gap2exp1:
5'-TTG AAG TGG AGC CGG ACG AG-3'
gap2exp2:
5'-CCT ATA GTA CGG CTG GCT GC-3'

The primers shown were synthesized by ARK Scientific GmbH Biosystems (Darmstadt, Germany) and the PCR reaction was carried out by the standard PCR method of Innis et al. (PCR protocols. A Guide to Methods and Applications, 1990, Academic Press) with Pwo-Polymerase from Roche Diagnostics GmbH (Mannheim, Germany). With the aid of the polymerase chain reaction, the primers allow amplification of a DNA fragment 2022 bp in size, which carries the gap2 gene with the potential promoter region. The DNA sequence of the amplified DNA fragment was checked by sequencing.

The amplified DNA fragment was ligated with the Zero Blunt™ Kit of Invitrogen Corporation(Carlsbad, CA, USA; Catalogue Number K2700-20) in den vector pCR®Blunt II (Bernard et al., Journal of Molecular Biology, 234: 534-541 (1993)).

The E. coli strain TOP10 was then electroporated with the ligation batch (Hanahan, In: DNA cloning. A Practical Approach. Vol. I, IRL-Press, Oxford, Washington DC, USA, 1985). Selection for plasmid-carrying cells was made by plating out the transformation batch on LB agar (Sambrook et al., Molecular Cloning: A Laboratory Manual. 2^{nd} Ed., Cold Spring Harbor Laboratory Press, Cold Spring Harbor, N.Y., 1989), which had been supplemented with 25 mg/l kanamycin. Plasmid DNA was isolated from a transformant with the aid of the QIAprep Spin Miniprep Kit from Qiagen and checked by restriction with the restriction enzyme EcoRI and subsequent agarose gel electrophoresis (0.8%). The plasmid was called pCRB1-gap2exp.

### 3.2 Preparation of the E. coli - C. glutamicum shuttle vector pEC-K18mob2

The E. coli - C. glutamicum shuttle vector was constructed according to the prior art. The vector contains the replication region rep of the plasmid pGA1 including the replication effector per (US-A- 5,175,108; Nesvera et al., Journal of Bacteriology 179, 1525-1532 (1997)), the kanamycin resistance-imparting aph(3')-IIa gene of the transposon Tn5 (Beck et al., Gene 19, 327-336 (1982)), the replication region oriV of the plasmid pMB1 (Sutcliffe, Cold Spring Harbor Symposium on Quantitative Biology 43, 77-90 (1979)), the lacZa gene fragment including the lac promoter and a multiple cloning site (mcs) (Norrander, J.M. et al., Gene 26, 101-106 (1983)) and the mob region of the plasmid RP4 (Simon et al., Bio/Technology 1:784-791 (1983)).

The vector pEC-K18mob2 constructed was transferred into C. glutamicum DSM5715 by means of electroporation (Liebl et al., 1989, FEMS Microbiology Letters, 53:299-303).

Selection of the transformants took place on LBHIS agar comprising 18.5 g/l brain-heart infusion broth, 0.5 M sorbitol, 5 g/l Bacto-tryptone, 2.5 g/l Bacto-yeast extract, 5 g/l NaCl and 18 g/l Bacto-agar, which had been supplemented with 25 mg/l kanamycin. Incubation was carried out for 2 days at 33°C.

Plasmid DNA was isolated from a transformant by conventional methods (Peters-Wendisch et al., 1998, Microbiology, 144, 915 -927), cleaved with the restriction endonucleases EcoRI and HindIII, and the plasmid was checked by subsequent agarose gel electrophoresis.

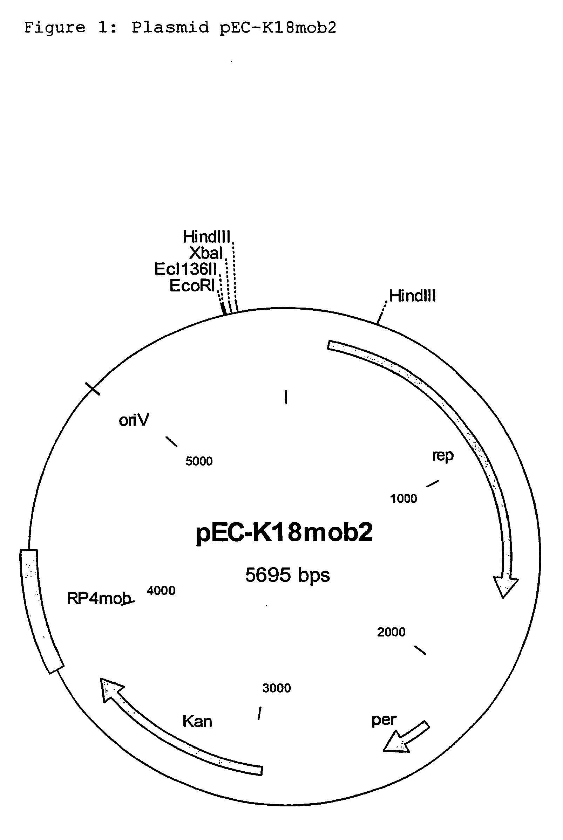

The plasmid construction thus obtained was called pECK18mob2 and is shown in figure 1. The strain obtained by electroporation of the plasmid pEC-K18mob2 in the C. glutamicum strain DSM5715 was called DSM5715/pEC-K18mob2 and deposited on 20th January 2000 as DSM13245 at the Deutsche Sammlung für Mikroorganismen und Zellkulturen (DSMZ = German Collection of Microorganisms and Cell Cultures, Braunschweig, Germany) in accordance with the Budapest Treaty.

### 3.3 Cloning of gap2 in the E. coli-C. glutamicum shuttle vector pEC-K18mob2

For cloning of the gap2 gene in the E. coli - C. glutamicum shuttle vector pEC-K18mob2 described in example 3.2, plasmid DNA of pEC-K18mob2 was cleaved completely with the restriction endonucleases Ec1136II and XbaI and treated with alkaline phosphatase (Alkaline Phosphatase, Roche Diagnostics GmbH, Mannheim, Germany).

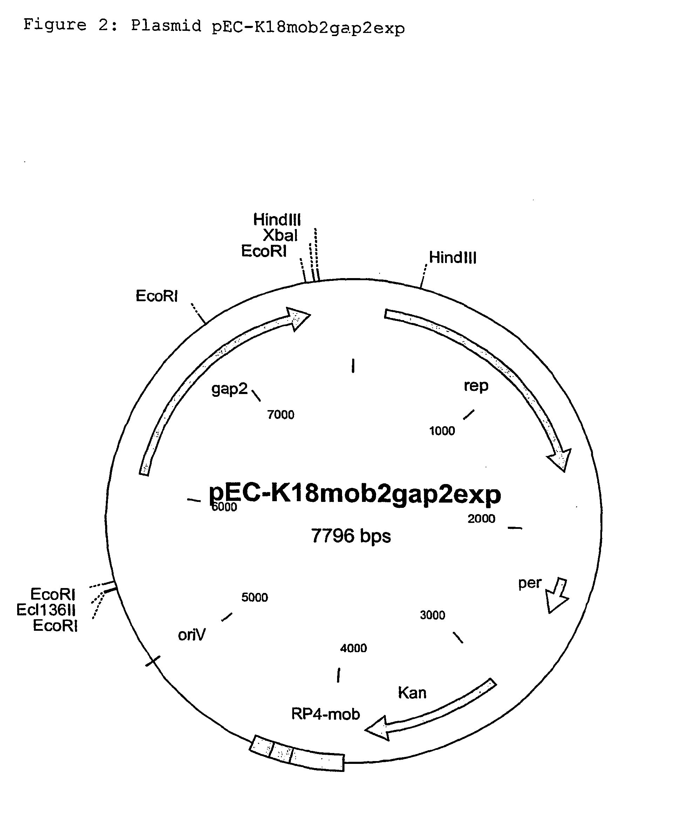

The vector pCRBl-gap2exp was isolated from Escherichia coli Top10 and cleaved completely with the restriction endonucleases Ecl136II and XbaI and the fragment approx. 2120 bp in size with the gap2 gene was purified from a 0.8% agarose gel (QIAquick Gel Extraction Kit der Firma Qiagen, Hilden, Germany). The fragment with the gap2 gene was then ligated with the vector pEC-K18mob2 (T4-Ligase, Roche Diagnostics GmbH, Mannheim; Germany). The ligation batch was transformed in the E. coli strain DH5alphamcr (Hanahan, In: DNA cloning. A Practical Approach. Vol. I, IRL-Press, Oxford, Washington DC, USA). Selection for plasmid-carrying cells was made by plating out the transformation batch on LB agar (Sambrook et al., Molecular Cloning: A Laboratory Manual. 2^{nd} Ed., Cold Spring Harbor Laboratory Press, Cold Spring Harbor, N.Y., 1989), which had been supplemented with 25 mg/l kanamycin. Plasmid DNA was isolated from a transformant with the aid of the QIAprep Spin Miniprep Kit from Qiagen (Hilden, Germany) and checked by treatment with the restriction enzyme EcoRI with subsequent agarose gel electrophoresis. The plasmid was called pEC-K18mob2gap2exp and is shown in figure 2.

### Example 4

### Transformation of the strain DSM5715 with the plasmid pECK18mob2gap2exp

The strain DSM5715 was transformed with the plasmid pECK18mob2gap2exp using the electroporation method described by Liebl et al., (FEMS Microbiology Letters, 53:299-303 (1989)). Selection of the transformants took place on LBHIS agar comprising 18.5 g/l brain-heart infusion broth, 0.5 M sorbitol, 5 g/l Bacto-tryptone, 2.5 g/l Bacto-yeast extract, 5 g/l NaCl and 18 g/l Bacto-agar, which had been supplemented with 25 mg/l kanamycin. Incubation was carried out for 2 days at 33°C.

Plasmid DNA was isolated from a transformant by conventional methods (Peters-Wendisch et al., 1998, Microbiology, 144, 915 -927), cleaved with the restriction endonuclease EcoRI, and the plasmid was checked by subsequent agarose gel electrophoresis. The strain obtained in this way was called DSM5715/pEC-K18mob2gap2exp.

### Example 5

### Preparation of Lysine

The C. glutamicum strain DSM5715/pEC-K18mob2gap2exp obtained in example 4 was cultured in a nutrient medium suitable for the production of lysine and the lysine content in the culture supernatant was determined.

For this, the strain was first incubated on an agar plate with the corresponding antibiotic (brain-heart agar with kanamycin (25 mg/l)) for 24 hours at 33°C. Starting from this agar plate culture, a preculture was seeded (10 ml medium in a 100 ml conical flask). The complete medium CgIII was used as the medium for the preculture.

| Medium Cg III | |
|---|---|
| NaCl | 2.5 g/l |
| Bacto-Peptone | 10 g/l |
| Bacto-Yeast extract | 10 g/l |
| Glucose (autoclaved separately) | 2% (w/v) |
| The pH was brought to pH 7.4 | |

Kanamycin (25 mg/l) was added to this. The preculture was incubated for 16 hours at 33°C at 240 rpm on a shaking machine. A main culture was seeded from this preculture such that the initial OD (660nm) of the main culture was 0.05. Medium MM was used for the main culture.

| Medium MM | |
|---|---|
| CSL (corn steep liquor) | 5 g/l |
| MOPS (morpholinopropanesulfonic acid) | 20 g/l |
| Glucose (autoclaved separately) | 100 g/l |
| (NH₄)₂SO₄ | 25 g/l |
| KH₂PO₄ | 0.1 g/l |
| MgSO₄ * 7 H₂O | 1.0 g/l |
| CaCl₂ * 2 H₂O | 10 mg/l |
| FeSO₄ * 7 H₂O | 10 mg/l |
| MnSO₄ * H₂O | 5.0 mg/l |
| Biotin (sterile-filtered) | 0.3 mg/l |
| Thiamine * HCl (sterile-filtered) | 0.2 mg/l |
| L-Leucine (sterile-filtered) | 0.1 g/l |
| CaCO₃ | 25 g/l |

The CSL, MOPS and the salt solution were brought to pH 7 with aqueous ammonia and autoclaved. The sterile substrate and vitamin solutions were then added, as well as the CaCO₃ autoclaved in the dry state.

Culturing is carried out in a 10 ml volume in a 100 ml conical flask with baffles. Kanamycin (25 mg/l) was added. Culturing was carried out at 33°C and 80% atmospheric humidity.

After 72 hours, the OD was determined at a measurement wavelength of 660 nm with a Biomek 1000 (Beckmann Instruments GmbH, Munich). The amount of lysine formed was determined with an amino acid analyzer from Eppendorf-BioTronik (Hamburg, Germany) by ion exchange chromatography and post-column derivation with ninhydrin detection.

The result of the experiment is shown in table 1.

**Table 1**

| Strain | OD (660 nm) | Lysine HCl g/l |
|---|---|---|
| DSM5715 | 11.8 | 14.43 |
| DSM5715/pEC-K18mob2gap2exp | 12.1 | 15.21 |

### Brief Description of the Figures:

Figure 1: Map of the plasmid pEC-K18mob2
Figure 2: Map of the plasmid pEC-K18mob2gap2exp

The abbreviations and designations used have the following meaning:
- Kan:: Resistance gene for kanamycin
- per:: Gene for control of the number of copies from PGA1
- oriV:: ColE1-similar origin from pMB1
- rep:: Plasmid-coded replication region from C. glutamicum plasmid pGA1
- RP4mob:: RP4 mobilization site
- gap2:: gap2 gene from C. glutamicum
- EcoRI:: Cleavage site of the restriction enzyme EcoRI
- HindIII:: Cleavage site of the restriction enzyme HindIII
- Ecl13II:: Cleavage site of the restriction enzyme Ecl136II
- XbaI:: Cleavage site of the restriction enzyme XbaI

## Claims

1. An isolated coryneform bacterium wherein a polynucleotide encoding a polypeptide having an amino acid sequence which is at least 90 % identical to the amino acid sequence set out in SEQ ID NO:2 is recombinantly overexpressed, wherein the polypeptide has the activity of glyceraldehyde-3-phosphate dehydrogenase 2 and said recombinant bacterium produces L-lysine in an improved manner compared to the non-recombinant L-lysine producing coryneform bacterium.

2. The Corynebacterium according to claim 1, wherein said polypeptide has an amino acid sequence which is at least 95 % identical to the amino acid sequence set out in SEQ ID NO:2.

3. The Corynebacterium according to claim 1 or 2, wherein said polynucleotide is chosen from the group:
a) polynucleotide having the nucleotide sequence as set out in SEQ ID NO:1; or
b) polynucleotides having nucleotide sequences corresponding to sequence SEQ ID NO:1 within the degeneracy of the genetic code; or
c) polynucleotides having nucleotide sequences with neutral sense mutations in SEQ ID NO:1.

4. The Corynebacterium of claim 1, wherein said polypeptide has the amino acid sequence set out in SEQ ID NO:2.

5. A process for the preparation of L-lysine, comprising the Corynebacterium according to claims 1 to 3.

6. A process for the preparation of L-lysine comprising:
fermentation of coryneform bacterium known to be able to produce said L-amino acid, wherein a polynucleotide encoding a polypeptide having an amino acid sequence which is at least 90 % identical to the amino acid sequence set out in SEQ ID NO:2 is recombinantly overexpressed wherein the polypeptide has the activity of glyceraldehyde-3-phosphate dehydrogenase 2 and said bacterium produces L-lysine in an improved manner.

7. The process according to claim 6, wherein said polypeptide is identical to the polypeptide having the amino acid sequence set out in SEQ ID NO:2.

8. The process according to claim 6 or 7, wherein said polynucleotide is chosen from the group:
a) polynucleotide having the nucleotide sequence as set out in SEQ ID NO:1;
b) polynucleotides having nucleotide sequences corresponding to sequence SEQ ID NO:1 within the degeneracy of the genetic code; or
c) polynucleotides having nucleotide sequences with neutral sense mutations in SEQ ID NO:1

9. The process according to claims 6 to 8
a) fermentation of said Corynebacterium
b) concentration of L-lysine in the medium or in the cells of the bacteria and
c) isolation of said L-amino acid.

10. The process according to claims 6 to 9, wherein coryneform bacteria are transformed with a plasmid vector and the plasmid vector carries said nucleotide sequences.

11. The process according to claim 9, wherein coryneform bacteria are fermented in which at the same time one or more of the genes are overexpressed chosen from the group consisting of
a) the dapA gene which codes for dihydrodipicolinate synthase,
b) the gap gene which codes for glyceraldehyde 3-phosphate dehydrogenase,
c) the tpi gene which codes for triose phosphate isomerase,
d) the pgk gene which codes for 3-phosphoglycerate kinase,
e) the zwf gene which codes for glucose 6-phosphate dehydrogenase,
f) the pyc gene which codes for pyruvate carboxylase,
g) the mqo gene which codes for malate-quinone oxidoreductase,
h) the lysC gene which codes for a aspartate kinase,
i) the lysE gene which codes for a protein for lysine export,
j) the hom gene which codes for homoserine dehydrogenase
k) the ilvA gene which codes for threonine dehydratase,
1) the ilvBN gene which codes for acetohydroxy-acid synthase,
m) the ilvD gene which codes for dihydroxy-acid dehydratase.

12. The process according to claim 9, wherein coryneform bacteria are fermented in which at the same time one or more of the genes chosen from the group consisting of
a) the pck gene which codes for phosphoenol pyruvate carboxykinase,
b) the pgi gene which codes for glucose 6-phosphate isomerase,
c) the poxB gene which codes for pyruvate oxidase,
is or are attenuated.

13. Coryneform bacteria transformed with a vector which carries a polynucleotide according to claims 1 or 3.

14. The process according to one or more of claims 6 - 13, wherein microorganisms of the species Corynebacterium glutamicum are employed.

15. The process according to claim 15, wherein the Corynebacterium glutamicum strain DSM5715/pEC-K18mob2gap2exp is employed.

16. Escherichia coli strain DH5αmcr/pEC-K18mob2gap2exp as DSM 14407 deposited at the Deutsche Sammlung für Mikroorganismen und Zellkulturen [German Collection of Microorganisms and Cell Cultures], Braunschweig, Germany.

## Patentansprüche

1. Isoliertes coryneformes Bakterium, in dem ein für ein Polypeptid mit einer Aminosäuresequenz, die mindestens zu 90% mit der in SEQ ID NO:2 angegebenen Aminosäuresequenz identisch ist, codierendes Polynukleotid rekombinant überexprimiert wird, wobei das Polypeptid die Aktivität einer Glycerinaldehyd-3-phosphat-Dehydrogenase 2 aufweist und das rekombinante Bakterium L-Lysin auf verbesserte Weise im Vergleich zu dem nicht rekombinanten, L-Lysin produzierenden coryneformen Bakterium produziert.

2. Corynebacterium gemäß Anspruch 1, wobei das Polypeptid eine Aminosäuresequenz aufweist, die mindestens zu 95% mit der in SEQ ID NO:2 angegebenen Aminosäuresequenz identisch ist.

3. Corynebacterium gemäß Anspruch 1 oder 2, wobei das Polynukleotid ausgewählt ist aus der Gruppe:
a) Polynukleotid mit der wie in SEQ ID NO:1 angegebenen Nukleotidsequenz; oder
b) Polynukleotide mit Nukleotidsequenzen, die der Sequenz SEQ ID NO:1 im Rahmen der Degeneriertheit des genetischen Codes entsprechen; oder
c) Polynukleotide mit Nukleotidsequenzen mit neutralen Sense-Mutationen in SEQ ID NO:1.

4. Corynebacterium nach Anspruch 1, wobei das Polypeptid die in SEQ ID NO:2 angegebene Aminosäuresequenz aufweist.

5. Verfahren zur Herstellung von L-Lysin, umfassend das Corynebacterium gemäß den Ansprüchen 1 bis 3.

6. Verfahren zur Herstellung von L-Lysin, bei dem man
die Fermentation eines coryneformen Bakteriums durchführt, von dem man weiß, daß es zur Produktion dieser L-Aminosäure fähig ist, und in dem ein für ein Polypeptid mit einer Aminosäuresequenz, die mindestens zu 90% mit der in SEQ ID NO:2 angegebenen Aminosäuresequenz identisch ist, codierendes Polynukleotid rekombinant überexprimiert wird, wobei das Polypeptid die Aktivität einer Glycerinaldehyd-3-phosphat-Dehydrogenase 2 aufweist und das Bakterium auf verbesserte Weise L-Lysin produziert.

7. Verfahren gemäß Anspruch 6, wobei das Polypeptid zu dem Polypeptid mit der in SEQ ID NO:2 angegebenen Aminosäuresequenz identisch ist.

8. Verfahren gemäß Anspruch 6 oder 7, wobei das Polynukleotid ausgewählt ist aus der Gruppe:
a) Polynukleotid mit der wie in SEQ ID NO:1 angegebenen Nukleotidsequenz;
b) Polynukleotide mit Nukleotidsequenzen, die der Sequenz SEQ ID NO:1 im Rahmen der Degeneriertheit des genetischen Codes entsprechen; oder
c) Polynukleotide mit Nukleotidsequenzen mit neutralen Sense-Mutationen in SEQ ID NO:1.

9. Verfahren gemäß den Ansprüchen 6 bis 8, bei dem man
a) eine Fermentation des Corynebacterium,
b) eine Anreicherung von L-Lysin im Medium oder in den Zellen der Bakterien und
c) eine Isolierung der L-Aminosäure durchführt.

10. Verfahren gemäß den Ansprüchen 6 bis 9, wobei coryneforme Bakterien mit einem Plasmidvektor transformiert werden und der Plasmidvektor die Nukleotidsequenzen trägt.

11. Verfahren gemäß Anspruch 9, wobei man coryneforme Bakterien fermentiert, in denen gleichzeitig eines oder mehrere der Gene, ausgewählt aus der Gruppe:
a) das für die Dihydrodipicolinat-Synthase codierende dapA-Gen,
b) das für die Glycerinaldehyd-3-phosphat-Dehydrogenase codierende gap-Gen,
c) das für die Triosephosphat-Isomerase codierende tpi-Gen,
d) das für die 3-Phosphoglycerat-Kinase codierende pgk-Gen,
e) das für die Glucose-6-phosphat-Dehydrogenase codierende zwf-Gen,
f) das für die Pyruvat-Carboxylase codierende pyc-Gen,
g) das für die Malat:Chinon-Oxidoreduktase codierende mqo-Gen,
h) das für eine Aspartatkinase codierende lysC-Gen,
i) das für ein Protein für den Lysin-Export codierende lysE-Gen,
j) das für die Homoserin-Dehydrogenase codierende hom-Gen,
k) das für die Threonin-Dehydratase codierende ilvA-Gen,
1) das für die Acetohydroxysäure-Synthase codierende ilvBN-Gen,
m) das für die Dihydroxysäure-Dehydratase codierende ilvD-Gen,
überexprimiert sind.

12. Verfahren nach Anspruch 9, wobei man coryneforme Bakterien fermentiert, in denen gleichzeitig eines oder mehrere der Gene, ausgewählt aus der Gruppe:
a) das für die Phosphoenolpyruvat-Carboxykinase codierende pck-Gen,
b) das für die Glucose-6-phosphat-Isomerase codierende pgi-Gen,
c) das für die Pyruvat-Oxidase codierende poxB-Gen,
abgeschwächt ist bzw. sind.

13. Coryneforme Bakterien, transformiert mit einem Vektor, der ein Polynukleotid gemäß den Ansprüchen 1 oder 3 trägt.

14. Verfahren gemäß einem oder mehreren der Ansprüche 6 bis 13, wobei Mikroorganismen der Spezies Corynebacterium glutamicum eingesetzt werden.

15. Verfahren gemäß Anspruch 15, wobei der Corynebacterium glutamicum-Stamm DSM5715/pEC-K18mob2gap2exp eingesetzt wird.

16. Escherichia coli-Stamm DH5amcr/pEC-K18mob2gap2exp, hinterlegt unter DSM 14407 bei der Deutschen Sammlung für Mikroorganismen und Zellkulturen, Braunschweig.

## Revendications

1. Bactérie coryneforme isolée, dans laquelle un polynucléotide codant pour un polypeptide ayant une séquence d'acides aminés qui est identique au moins à 90% à la séquence d'acides aminés représentée par la SEQ ID NO:2 est surexprimé de manière recombinante, le polypeptide ayant l'activité de la glycéraldéhyde-3-phosphate déshydrogénase 2 et ladite bactérie recombinante produisant de la L-lysine de manière améliorée en comparaison de la bactérie coryneforme non recombinante productrice de L-lysine.

2. Corynebacterium selon la revendication 1, dans lequel ledit polypeptide a une séquence d'acides aminés qui est identique au moins à 95% à la séquence d'acides aminés représentée par la SEQ ID NO:2.

3. Corynebacterium selon la revendication 1 ou 2, dans lequel ledit polynucléotide est choisi dans le groupe constitué par :
a) un polynucléotide ayant la séquence nucléotidique représentée par la SEQ ID NO:1; ou
b) des polynucléotides ayant des séquences nucléotidiques correspondant à la séquence SEQ ID NO:1 dans le cadre de la dégénérescence du code génétique; ou
c) des polynucléotides ayant des séquences nucléotidiques présentant des mutations sens neutres de la SEQ ID NO:1.

4. Corynebacterium selon la revendication 1, dans lequel ledit polypeptide a la séquence d'acides aminés représentée par la SEQ ID NO:2.

5. Procédé pour la préparation de L-lysine, comprenant le Corynebacterium selon les revendications 1 à 3.

6. Procédé de préparation de L-lysine comprenant :
la fermentation de la bactérie coryneforme connue pour être à même de produire ledit acide aminé, dans laquelle un polynucléotide codant pour un polypeptide ayant une séquence d'acides aminés qui est identique au moins à 90% à la séquence d'acides aminés représentée par la SEQ ID NO:2, est surexprimé de manière recombinante, le polypeptide ayant l'activité de la glycéraldéhyde-3-phosphate déshydrogénase 2 et ladite bactérie produisant de la L-lysine de manière améliorée.

7. Procédé selon la revendication 6, dans lequel ledit polypeptide est identique au polypeptide ayant la séquence d'acides aminés représentée par la SEQ ID NO:2.

8. Procédé selon la revendication 6 ou 7, dans lequel ledit polynucléotide est choisi dans le groupe constitué par les suivants :
a) un polynucléotide ayant la séquence nucléotidique représentée par la SEQ ID NO:1;
b) des polynucléotides ayant des séquences nucléotidiques correspondant à la séquence SEQ ID NO:1 dans le cadre de la dégénérescence du code génétique; ou
c) des polynucléotides ayant des séquences nucléotidiques présentant des mutations sens neutres de la SEQ ID NO:1.

9. Procédé selon les revendications 6 à 8 comprenant :
a) la fermentation dudit Corynebacterium,
b) la concentration de la L-lysine dans le milieu ou dans les cellules bactériennes et
c) l'isolement dudit acide aminé L.

10. Procédé selon les revendications 6 à 9, dans lequel les bactéries coryneformes sont transformées avec un vecteur plasmidique, le vecteur plasmidique portant lesdites séquences nucléotidiques.

11. Procédé selon la revendication 9, dans lequel on fait fermenter les bactéries coryneformes dans lesquelles, en même temps, on surexprime un ou plusieurs gènes choisis dans le groupe constitué des suivants :
a) le gène dapA qui code pour la dihydrodipicolinate synthase,
b) le gène gap qui code pour la glycéraldéhyde 3-phosphate déshydrogénase,
c) le gène tpi qui code pour la triose phosphate isomérase,
d) le gène pgk qui code pour la 3-phospho-glycérate kinase,
e) le gène zwf qui code pour la glucose 6-phosphate déshydrogénase,
f) le gène pyc qui code pour la pyruvate carboxylase,
g) le gène mqo qui code pour la malate-quinone oxydoréductase,
h) le gène lysC qui code pour une aspartate kinase,
i) le gène lysE qui code pour une protéine d'export de la lysine,
j) le gène hom qui code pour l'homosérine déshydrogénase,
k) le gène ilvA qui code pour la thréonine déshydratase,
l) le gène ilvBN qui code pour l'acétohydroxyacide synthase, et
m) le gène ilvD qui code pour la dihydroxy-acide déshydratase.

12. Procédé selon la revendication 9, dans lequel on fait fermenter les bactéries coryneformes dans lesquelles, en même temps, on atténue un ou plusieurs gènes choisis dans le groupe constitué des suivants :
a) le gène pck qui code pour la phosphoénol pyruvate carboxykinase,
b) le gène pgi qui code pour la glucose 6-phosphate isomérase, et
c) le gène poxB qui code pour la pyruvate oxydase.

13. Bactéries coryneformes transformées avec un vecteur qui porte un polynucléotide selon la revendication 1 ou 3.

14. Procédé selon l'une ou plusieurs des revendications 6 à 13, dans lequel on utilise des microorganismes de l'espèce Corynebacterium glutamicum.

15. Procédé selon la revendication 15, dans lequel on utilise la souche DSM5715/pEC-K18mob2gap2exp de Corynebacterium glutamicum.

16. Souche DH5αmcr/pEC-K18mob2gap2exp d'Escherichia coli, désignée par DSM 14407, déposée auprès de la Deutsche Sammlung für Mikroorganismen und Zellkulturen [German Collection of Microorganisms and Cell Cultures], Braunschweig, Allemagne.


### SEQUENCE PROTOCOL

<110> Degussa AG
<120> Nucleotide sequences which code for the gap2 gene
<130> 000463 BT
<140>
   <141>
<160> 4
<170> PatentIn Ver. 2.1
<210> 1
   <211> 1839
   <212> DNA
   <213> Corynebacterium glutamicum
<220>
   <221> CDS
   <222> (200)..(1639)
   <223> gap2 gene
<400> 1
   agggaactgg gattacgcct actgcctggt aggagtcggc ctggaatcga ttgcgaaggg 60
   tagtgcaaag cagatactgg aatcattaac accttccgct ttgggctaat gttgggggga 120
   gtgctttcaa ctatccacga gagctgccca gtgataaacc ccgggttaac cccacgccta 180
   agtcagtgaa ggacttttt atg acg cac aac cac aag gac tgg aac gat cgc  232
                       Met Thr His Asn His Lys Asp Trp Asn Asp Arg
                        1               5                   10
   att gca gtt gcg gag gaa atg gtg ccg ttg atc ggg cgc ctg cac cgc    280
   Ile Ala Val Ala Glu Glu Met Val Pro Leu Ile Gly Arg Leu His Arg
               15                  20                  25
   aac aac aac gtg gtg gtt tcc gta ttc ggt cgt ctc ctt gtg aat gtc    328
   Asn Asn Asn Val Val Val Ser Val Phe Gly Arg Leu Leu Val Asn Val
           30                  35                  40
   tca gac atc gat atc atc aag tct cac cgc tac gcc cgc cac atc ata    376
   Ser Asp Ile Asp Ile Ile Lys Ser His Arg Tyr Ala Arg His Ile Ile
       45                  50                  55
   tcc aag gaa ctt cca ctg gaa agc tcc ttg gat att ttg cgc gaa ctg    424
   Ser Lys Glu Leu Pro Leu Glu Ser Ser Leu Asp Ile Leu Arg Glu Leu
   60                  65                  70                  75
   gta gat atg aac ctt ggt acc gca tcg atc gac ctg gga cag ctg gcc    472
   Val Asp Met Asn Leu Gly Thr Ala Ser Ile Asp Leu Gly Gln Leu Ala
                   80                  85                  90
   tac agc ttc gaa gaa tcc gaa agc acc gac ctg cgt gcc ttc ctg gag    520
   Tyr Ser Phe Glu Glu Ser Glu Ser Thr Asp Leu Arg Ala Phe Leu Glu
               95                  100                 105
   gac gct ctc gcg ccg gtc att ggt gcg gaa acc gac atc aac cca act    568
   Asp Ala Leu Ala Pro Val Ile Gly Ala Glu Thr Asp Ile Asn Pro Thr
           110                 115                 120
   gat atc gtg ctg tac ggt ttc ggc cgc atc ggt cgc ctg ctg gcc cgc    616
   Asp Ile Val Leu Tyr Gly Phe Gly Arg Ile Gly Arg Leu Leu Ala Arg
       125                 130                 135
   atc ctg gtt tcc cgc gag gca ctg tat gac ggt gct cgt ctg cgc gcc    664
   Ile Leu Val Ser Arg Glu Ala Leu Tyr Asp Gly Ala Arg Leu Arg Ala
   140                 145                 150                 155
   atc gtg gtc cgc aaa aat ggt gaa gaa gac ctg gtc aag cgc gca tcc    712
   Ile Val Val Arg Lys Asn Gly Glu Glu Asp Leu Val Lys Arg Ala Ser
                   160                 165                 170
   ttg ctg cgt cgt gat tct gtc cac ggt gga ttc gat ggc acc atc acc    760
   Leu Leu Arg Arg Asp Ser Val His Gly Gly Phe Asp Gly Thr Ile Thr
               175                 180                 185
   acc gat tat gac aac aac atc atc tgg gcc aac ggc acc cca atc aag    808
   Thr Asp Tyr Asp Asn Asn Ile Ile Trp Ala Asn Gly Thr Pro Ile Lys
           190                 195                 200
   gtc atc tac tcc aat gac cca gcc acc att gat tac acc gaa tac ggc    856
   Val Ile Tyr Ser Asn Asp Pro Ala Thr Ile Asp Tyr Thr Glu Tyr Gly
       205                 210                 215
   atc aat gac gcc gtc gtg gta gac aac acc ggc cgc tgg cgt gac cgc    904
   Ile Asn Asp Ala Val Val Val Asp Asn Thr Gly Arg Trp Arg Asp Arg
   220                 225                 230                 235
   gaa ggc ctg tcc cag cac ctc aag tcc aag ggc gtt gcc aag gtt gta    952
   Glu Gly Leu Ser Gln His Leu Lys Ser Lys Gly Val Ala Lys Val Val
                   240                 245                 250
   ctc acc gcg ccg ggc aag ggc gat ctg aag aac atc gtg tac ggc atc    1000
   Leu Thr Ala Pro Gly Lys Gly Asp Leu Lys Asn Ile Val Tyr Gly Ile
               255                 260                 265
   aac cac acc gac atc acc gca gat gat cag atc gtt tcc gca gca tca    1048
   Asn His Thr Asp Ile Thr Ala Asp Asp Gln Ile Val Ser Ala Ala Ser
           270                 275                 280
   tgc acc acc aat gcc att acc cca gtg ctc aag gtg atc aat gat cgc    1096
   Cys Thr Thr Asn Ala Ile Thr Pro Val Leu Lys Val Ile Asn Asp Arg
       285                 290                 295
   tac ggc gtg gaa ttc ggc cac gta gaa acc gtt cac tcc ttc acc aat    1144
   Tyr Gly Val Glu Phe Gly His Val Glu Thr Val His Ser Phe Thr Asn
   300                 305                 310                 315
   gac cag aac ctg atc gac aac ttc cac aag ggt tct cgc cgt ggt cgc    1192
   Asp Gln Asn Leu Ile Asp Asn Phe His Lys Gly Ser Arg Arg Gly Arg
                   320                 325                 330
   gca gca ggt ctg aat atg gtt ctc acc gaa acc ggc gct gca aag gct    1240
   Ala Ala Gly Leu Asn Met Val Leu Thr Glu Thr Gly Ala Ala Lys Ala
               335                 340                 345
   gta tcc aag gcg ctt cca gag ctg gaa ggc aag ctc acc ggc aat gcc    1288
   Val Ser Lys Ala Leu Pro Glu Leu Glu Gly Lys Leu Thr Gly Asn Ala
           350                 355                 360
   atc cgc gtt cct acc cct gac gtg tcc atg gct gtg ctc aac ttg acc    1336
   Ile Arg Val Pro Thr Pro Asp Val Ser Met Ala Val Leu Asn Leu Thr
       365                 370                 375
   ctg aac acg gag gtg gac cgc gat gag gtc aac gag ttc ctc cgc cgt    1384
   Leu Asn Thr Glu Val Asp Arg Asp Glu Val Asn Glu Phe Leu Arg Arg
   380                 385                 390                 395
   gtg tcc ctg cac tct gac ttg cgc cag caa atc gac tgg atc cgt tcc    1432
   Val Ser Leu His Ser Asp Leu Arg Gln Gln Ile Asp Trp Ile Arg Ser
                   400                 405                 410
   cca gag gtt gtt tcc act gac ttc gtg ggc acc acc cac gcg ggc atc    1480
   Pro Glu Val Val Ser Thr Asp Phe Val Gly Thr Thr His Ala Gly Ile
               415                 420                 425
   gtt gat ggt cta gcc acc atc gca acc ggt cgc cac ctg gtg ctt tac    1528
   Val Asp Gly Leu Ala Thr Ile Ala Thr Gly Arg His Leu Val Leu Tyr
           430                 435                 440
   gtg tgg tac gac aac gag ttc ggc tac tcc aac cag gtc att cgc atc    1576
   Val Trp Tyr Asp Asn Glu Phe Gly Tyr Ser Asn Gln Val Ile Arg Ile
       445                 450                 455
   gtc gag gag atc gcc ggc gtg cgt cct cgc gtg tac ccg gag cgc agg    1624
   Val Glu Glu Ile Ala Gly Val Arg Pro Arg Val Tyr Pro Glu Arg Arg
   460                 465                 470                 475
   cag cca gcc gta cta taggttatcc aagcctaata cactacgatt aggcatatga    1679
   Gln Pro Ala Val Leu
                   480
   gtaagcgtaa gtcacgggtg aaagttaccc acaatccatt gggcatgaaa gtcaaagaca 1739
   cgtgctgtcg caaaacgccg cgctgcacca actgcccagt ggtgtacaca cgcttactca 1799
   aatccggggt cttagaaaac gacgacgccg acctgccgcg                       1839
<210> 2
   <211> 480
   <212> PRT
   <213> Corynebacterium glutamicum
<400> 2
   Met Thr His Asn His Lys Asp Trp Asn Asp Arg Ile Ala Val Ala Glu
    1               5                  10                  15
   Glu Met Val Pro Leu Ile Gly Arg Leu His Arg Asn Asn Asn Val Val
               20                  25                  30
   Val Ser Val Phe Gly Arg Leu Leu Val Asn Val Ser Asp Ile Asp Ile
           35                  40                  45
   Ile Lys Ser His Arg Tyr Ala Arg His Ile Ile Ser Lys Glu Leu Pro
       50                  55                  60
   Leu Glu Ser Ser Leu Asp Ile Leu Arg Glu Leu Val Asp Met Asn Leu
   65 70 75 80
   Gly Thr Ala Ser Ile Asp Leu Gly Gln Leu Ala Tyr Ser Phe Glu Glu
   85 90 95
   Ser Glu Ser Thr Asp Leu Arg Ala Phe Leu Glu Asp Ala Leu Ala Pro
   100 105 110
   Val Ile Gly Ala Glu Thr Asp Ile Asn Pro Thr Asp Ile Val Leu Tyr
   115 120 125
   Gly Phe Gly Arg Ile Gly Arg Leu Leu Ala Arg Ile Leu Val Ser Arg
   130 135 140
   Glu Ala Leu Tyr Asp Gly Ala Arg Leu Arg Ala Ile Val Val Arg Lys
   145 150 155 160
   Asn Gly Glu Glu Asp Leu Val Lys Arg Ala Ser Leu Leu Arg Arg Asp
   165 170 175
   Ser Val His Gly Gly Phe Asp Gly Thr Ile Thr Thr Asp Tyr Asp Asn
   180 185 190
   Asn Ile Ile Trp Ala Asn Gly Thr Pro Ile Lys Val Ile Tyr Ser Asn
   195 200 205
   Asp Pro Ala Thr Ile Asp Tyr Thr Glu Tyr Gly Ile Asn Asp Ala Val
   210 215 220
   Val Val Asp Asn Thr Gly Arg Trp Arg Asp Arg Glu Gly Leu Ser Gln
   225 230 235 240
   His Leu Lys Ser Lys Gly Val Ala Lys Val Val Leu Thr Ala Pro Gly
   245 250 255
   Lys Gly Asp Leu Lys Asn Ile Val Tyr Gly Ile Asn His Thr Asp Ile
   260 265 270
   Thr Ala Asp Asp Gln Ile Val Ser Ala Ala Ser Cys Thr Thr Asn Ala
   275 280 285
   Ile Thr Pro Val Leu Lys Val Ile Asn Asp Arg Tyr Gly Val Glu Phe
   290 295 300
   Gly His Val Glu Thr Val His Ser Phe Thr Asn Asp Gln Asn Leu Ile
   305 310 315 320
   Asp Asn Phe His Lys Gly Ser Arg Arg Gly Arg Ala Ala Gly Leu Asn
   325 330 335
   Met Val Leu Thr Glu Thr Gly Ala Ala Lys Ala Val Ser Lys Ala Leu
   340 345 350
   Pro Glu Leu Glu Gly Lys Leu Thr Gly Asn Ala Ile Arg Val Pro Thr
   355 360 365
   Pro Asp Val Ser Met Ala Val Leu Asn Leu Thr Leu Asn Thr Glu Val
   370 375 380
   Asp Arg Asp Glu Val Asn Glu Phe Leu Arg Arg Val Ser Leu His Ser
   385 390 395 400
   Asp Leu Arg Gln Gln Ile Asp Trp Ile Arg Ser Pro Glu Val Val Ser
                   405                 410                 415
   Thr Asp Phe Val Gly Thr Thr His Ala Gly Ile Val Asp Gly Leu Ala
               420                 425                 430
   Thr Ile Ala Thr Gly Arg His Leu Val Leu Tyr Val Trp Tyr Asp Asn
           435                 440                 445
   Glu Phe Gly Tyr Ser Asn Gln Val Ile Arg Ile Val Glu Glu Ile Ala
       450                 455                 460
   Gly Val Arg Pro Arg Val Tyr Pro Glu Arg Arg Gln Pro Ala Val Leu
   465                 470                 475                 480
<210> 3
   <211> 20
   <212> DNA
   <213> Corynebacterium glutamicum
<220>
   <223> Primer gap2exp1
<400> 3
   ttgaagtgga gccggacgag 20
<210> 4
   <211> 20
   <212> DNA
   <213> Corynebacterium glutamicum
<220>
   <223> Primer gap2exp2
<400> 4
   cctatagtac ggctggctgc 20